(19) Europäisches Patentamt
European Patent Office
Office européen des brevets

(11) Numéro de publication : **0 099 815**
**B1**

(12) # FASCICULE DE BREVET EUROPÉEN

(45) Date de publication du fascicule du brevet : **28.05.86**

(51) Int. Cl.[4] : **C 07 D401/04**, A 61 K 31/44

(21) Numéro de dépôt : **83401427.6**

(22) Date de dépôt : **11.07.83**

(54) **Dérivés du pyridyl-3 alcoxy-(où phénoxy- ou aralkyloxy-)5 pyrazole, procédé de préparation et application en thérapeutique.**

(30) Priorité : **12.07.82 FR 8212176**

(43) Date de publication de la demande : **01.02.84 Bulletin 84/05**

(45) Mention de la délivrance du brevet : **28.05.86 Bulletin 86/22**

(84) Etats contractants désignés : **AT BE CH DE FR GB IT LI LU NL SE**

(56) Documents cités : **FR-A- 2 167 997**

(73) Titulaire : **ETABLISSEMENTS NATIVELLE S.A.**
**27, rue de la Procession**
**F-75015 Paris (FR)**

(72) Inventeur : **Jarreau, François-Xavier**
**5, rue Louis Hervé**
**F-78000 Versailles (FR)**
Inventeur : **Koenig, Jean-Jacques**
**31, rue du Panorama**
**F-77672 Vernou-la-Celle s/Seine (FR)**

(74) Mandataire : **L'Helgoualch, Jean et al**
**OFFICE PICARD 134 Boulevard de Clichy**
**F-75018 Paris (FR)**

**EP 0 099 815 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention, réalisée dans les laboratoires du CERES — Centre Européen de Recherche Scientifique — concerne de nouveaux dérivés du pyrazole, et plus particulièrement de nouveaux dérivés du pyridyl-3 alcoxy (ou phénoxy- ou aralkyloxy)-5 pyrazole, un procédé pour leur préparation, ainsi que leur application en thérapeutique.

Dans le domaine des composés utilisables en thérapeutique, on connaît divers dérivés de pyridinone ou de pyridazinone substitués par un groupe pyridyle, et par exemple, des pyridyl-5 pyridinones-2 comme aux brevets français 2.327.779, 2.470.124 et 2.477.148, ou des (pyridyl-4')-6 (2H)-pyridazinones-3 comme dans la demande de brevet français 2.481.284. Ces composés ont la propriété d'augmenter la contractilité cardiaque et peuvent être utilisés comme médicaments cardiotoniques. Par contre, on ne connaît pas de dérivés comparables du pyrazole présentant de telles propriétés.

Par ailleurs, le brevet français 2.167.997 décrit des dérivés de l'amino-1 pyrazolone-5 et de l'amino-1 méthoxy-5 pyrazole possédant une activité dépressive sur le système nerveux central.

Les travaux réalisés par la demanderesse ont permis de montrer que certains dérivés de l'alcoxy-5 pyrazole, substitués en position 3 par un groupe pyridyle, possédaient d'intéressantes propriétés inotropes permettant leur application en thérapeutique comme médicaments cardiotoniques.

La présente invention a pour objet les produits nouveaux constitués par les dérivés du type pyridyl-3 alcoxy (ou phénoxy- ou aralkyloxy)-5 pyrazole, ainsi qu'un procédé pour la préparation de ces composés.

L'invention a également pour objet des compositions pharmaceutiques contenant comme principes actifs des dérivés du type pyridyl-3 alcoxy (ou phénoxy- ou aralkyloxy)-5 pyrazole utiles notamment comme agents cardiotoniques.

Les nouveaux dérivés du type pyridyl-3 alcoxy (ou phénoxy- ou aralkyloxy)-5 pyrazole conformes à la présente invention peuvent être représentés par la formule générale (I) ci-dessous :

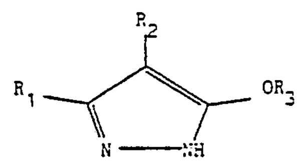

(I)

dans laquelle $R_1$ représente un groupe pyridyl-4, pyridyl-3 ou pyridyl-2 non substitué, ou substitué par un ou deux groupes alkyles inférieurs ; $R_2$ représente un atome d'hydrogène, un groupe alkyle inférieur et $R_3$ représente un groupe alkyle inférieur, un groupe phényle ou un groupe aralkyle.

L'expression « groupe alkyle inférieur » désigne un groupe alkyle linéaire ou ramifié, comportant 1 à 4 atomes de carbone, tel qu'un groupe méthyle, éthyle, n-propyle, ou isopropyle.

Lorsqu'il est substitué par un groupe alkyle inférieur, le groupe pyridyle peut être par exemple un groupe méthyl-2 pyridyl-4, méthyl-4 pyridyl-2, diméthyl-2,6 pyridyl-4, éthyl-2 pyridyl-4, isopropyl-2 pyridyl-4, ou diméthyl-2,6 pyridyl-3.

L'invention concerne de préférence les pyridyl-3 alcoxy (ou phénoxy- ou aralkyloxy)-5 pyrazoles de formule générale (I) ci-dessus dans laquelle $R_1$ est un groupe pyridyl-4, pyridyl-3 ou pyridyl-2, $R_2$ est un atome d'hydrogène ou un groupe méthyle, et $R_3$ est un groupe méthyle, éthyle, isopropyle, phényle ou benzyle.

Les composés de formule générale (I) peuvent exister sous plusieurs formes d'équilibre représentées ci-après :

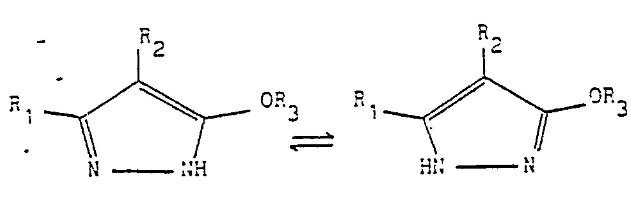

Bien entendu, l'invention concerne les diverses formes d'équilibre de ces composés.

L'invention s'étend également aux sels des pyridyl-3 alcoxy (ou phénoxy- ou aralkyloxy)-5 pyrazole de formule générale (I), et notamment les sels pharmaceutiquement acceptables, obtenus par action d'un acide minéral ou organique, suivant les méthodes usuelles de la technique. L'acide utilisé peut être choisi parmi l'acide chlorhydrique, l'acide sulfurique, l'acide oxalique, l'acide tartrique, l'acide fumarique, l'acide lactique, l'acide citrique, l'acide phosphorique, l'acide p-toluène sulfonique, l'acide formique, l'acide bromhydrique, l'acide maléique, l'acide sulfamique.

Les pyridyl-3 alcoxy (ou phénoxy- ou aralkyloxy)-5 pyrazoles de formule générale (I) peuvent être préparés conformément à l'invention par réaction des pyridyl-3 pyrazolones-5 de formule générale (II) :

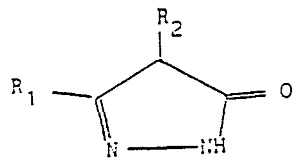

dans laquelle $R_1$ et $R_2$ ont la même signification que dans la formule (I), avec un agent d'alkylation dans un solvant organique approprié.

L'alkylation s'effectue au moyen d'un agent de O-alkylation, par exemple le diazométhane en solution, à température ambiante.

Le solvant peut être par exemple le benzène, le toluène, un alcool tel que le méthanol, l'éthanol ou l'isopropanol, un éther tel que l'éther diméthylique, l'éther diéthylique, le dioxanne, le tétrahydrofuranne, isolément ou en mélange.

Il peut être avantageux d'effectuer la réaction d'alkylation de la pyridyl-3 pyrazolone-5 de formule (II), suivant l'invention, par un alcool activé au moyen de triphénylphosphine et d'azodicarboxylate d'éthyle, en milieu neutre, par application de la méthode de O. Mitsunobu. Cette réaction d'alkylation s'effectue en ajoutant la triphénylphosphine à un mélange de pyridyl-3 pyrazolone-5 de formule (II), d'alcool et d'azodicarboxylate d'éthyle dans un solvant aprotique tel que le tétrahydrofuranne, le diméthylformamide ou un hydrocarbure aromatique, à une température comprise entre 0 et 20 °C. Inversement, on peut procéder en ajoutant l'azodicarboxylate d'éthyle à un mélange de pyridyl-3 pyrazolone-5, d'alcool et de triphénylphosphine. Suivant une variante, on peut ajouter la pyridyl-3 pyrazolone-5 et l'alcool à un mélange de triphénylphosphine et d'azodicarboxylate d'éthyle préalablement refroidi à une température comprise entre — 20° et — 10 °C.

L'alcool utilisé pour cette réaction est choisi en fonction du substituant que l'on veut greffer sur l'atome d'oxygène du noyau pyrazolone, et peut être représenté par la formule $R_3$—OH où $R_3$ est défini comme ci-dessus. On peut par exemple utiliser le méthanol, l'éthanol, l'isopropanol, etc.

Certaines pyridyl-3 pyrazolones-5 de formule générale (II) sont des produits connus, et par exemple la (pyridyl-4)-3 pyrazolone-5 est décrite par H.L. Yale et al. JACS 75, p. 1933 (1953) qui l'ont préparée par réaction de l'hydrate d'hydrazine sur l'isonicotinyl-acétate d'éthyle dans le n-propanol. Les composés de formule (II) peuvent être préparés par la même méthode, en changeant le produit de départ, par analogie, c'est-à-dire que l'on fait réagir un cétoester de formule (III)

$$R_1—CO—CHR_2—COOC_2H_5 \quad (III)$$

sur l'hydrate d'hydrazine pour obtenir les dérivés de formule (II) où $R_1$ et $R_2$ ont les définitions indiquées ci-dessus, par réaction de cyclisation, en milieu acide.

Les exemples suivants illustrent l'invention sans en limiter la portée. Les structures des dérivés ont été confirmées par l'analyse et les spectres IR et de RMN.

## Exemple 1

(pyridyl-4)-3 méthoxy-5 pyrazole

Dans un ballon de 1 litre contenant 16,1 g de (pyridyl-4)-3 pyrazolone-5 dans 150 ml de méthanol, à froid et sous agitation, on verse progressivement 300 ml d'une solution environ 0,5 N de diazométhane dans l'éther diéthylique, préparée sur place.

On laisse la réaction s'effectuer pendant 24 heures à température ambiante, puis on élimine le solvant par évaporation à sec. Le résidu est dilué dans 50 ml de chloroforme puis filtré pour éliminer la quantité restante de pyrazolone de départ qui n'a pas réagi, et on effectue une extraction de la phase chloroformique par de la soude diluée. On ajoute de l'acide chlorhydrique dilué pour ramener le pH à 4, et on extrait par le chloroforme, puis on fait cristalliser dans l'éther diisopropylique.

On obtient ainsi 9,1 g (rendement 52 %) de (pyridyl-4)-3 méthoxy-5 pyrazole. La pyrazolone de départ, récupérée par filtration de la solution chloroformique, peut être recyclée pour améliorer le rendement.

Point de fusion F = 140 °C
Spectre IR (Nujol) $\nu$ = 3 500 à 2 200, 1 610, 1 575, 1 550, 1 520, 1 400, 1 040 $cm^{-1}$
Spectre de RMN $\delta$ = 3,9(s, 3H) 6,4(s, 1H) 7,7 (2H) 8,6 (2H) 12,7 (1H mobile) ppm ($DMSOd_6$)

Chlorhydrate

A une solution de 50 ml d'isopropanol contenant 5 g de (pyridyl-4)-3 méthoxy-5 pyrazole, on ajoute lentement de l'acide chlorhydrique concentré jusqu'à ce que le pH soit d'environ 2 à 3. Les cristaux de chlorhydrate précipitent et sont recueillis par filtration (rendement 95 %).

Point de fusion F = 205 °C

## Exemple 2

(pyridyl-4)-3 méthyl-4 méthoxy-5 pyrazole

On place 1,5 g de (pyridyl-4)-3 méthyl-4 pyrazolone-5, 2,3 g de triphénylphosphine et 0,27 g de méthanol dans 30 ml de tétrahydrofuranne. On refroidit à 2 °C sur bain de glace et on ajoute goutte à goutte 1,6 g d'azodicarboxylate d'éthyle dissous dans 9 ml de tétrahydrofuranne.

Après l'addition, on laisse le milieu réactionnel revenir à température ambiante, puis, après 4 heures on filtre l'insoluble. On ajoute de l'acide chlorhydrique concentré sous agitation et on filtre le chlorhydrate de (pyridyl-4)-3 méthyl-4 méthoxy-5 pyrazole que l'on purifie par cristallisation dans le méthanol.

Point de fusion F = 263 °C
La base est extraite et obtenue avec un rendement de 40 % (0,65 g)
Point de fusion F = 140 °C
Spectre IR (Nujol) $\nu$ = 3 500 à 2 500 (max. à 3 300 et 3 150), 1 615, 1 590, 1 550, 1 515, 1 410, 1 170, 935, 830, 715 $cm^{-1}$

## Exemple 3

(pyridyl-3)-3 méthyl-4 méthoxy-5 pyrazole

On procède comme dans l'exemple 1 à partir de la (pyridyl-3)-3 méthyl-4 pyrazolone-5 pour obtenir le produit recherché avec un rendement de 45 %.

Point de fusion F = 134 °C
Spectre IR (Nujol) $\nu$ = 3 175, 1 510, 1 415, 1 170, 1 020 $cm^{-1}$
Chlorhydrate :
Point de fusion F = 212-214 °C.

## Exemple 4

(pyridyl-2)-3 méthyl-4 méthoxy-5 pyrazole

On procède comme dans l'exemple 1 à partir de la (pyridyl-2)-3 méthyl-4 pyrazole-5 pour obtenir le produit recherché avec un rendement de 40 %.

Chlorhydrate :
Point de fusion F = 156-158 °C

## Exemple 5

(pyridyl-4)-3 benzyl-4 méthoxy-5 pyrazole

On fait agir 0,8 g de diazométhane sur 3,6 g de (pyridyl-4)-3 benzyl-4 pyrazolone-5 suivant la technique décrite dans l'exemple 1, dans un mélange de tétrahydrofuranne et de méthanol, pendant 24 heures.

Après évaporation du solvant, extraction par le chlorure de méthylène, lavage et trituration dans l'éther diisopropylique, on obtient 2,2 g (rendement 60 %) de cristaux de (pyridyl-4)-3 benzyl-4 méthoxy-5 pyrazole purifiés par précipitation du chlorhydrate dans l'isopropanol.

Spectre IR (Nujol) $\nu$ = 3 380, 3 160, 1 635, 1 605, 1 580, 1 505, 1 490 $cm^{-1}$.
Chlorhydrate :
Point de fusion F = 186 °C (dec.)

## Exemple 6

(pyridyl-4)-3 méthyl-4 éthoxy-5 pyrazole

On place, sous agitation, 5,3 g de pyridyl-3 méthyl-4 pyrazolone-5 dans 120 ml de tétrahydrofuranne, 8,6 g de triphénylphosphine et 1,8 ml d'éthanol. On refroidit sur bain de glace à 2 °C et on ajoute goutte à goutte 5,2 ml d'azodicarboxylate d'éthyle en solution dans 20 ml de tétrahydrofuranne. On laisse réagir 4 heures à température ambiante.

Le précipité de pyrazolone qui n'a pas réagi est filtré. Le tétrahydrofuranne est évaporé à sec, et on reprend le résidu avec une solution d'acide chlorhydrique. Après lavage au chlorure de méthylène, le

chlorhydrate de (pyridil-4)-3 méthyl-4 éthoxy-5 pyrazole obtenu est recristallisé dans le méthanol, puis transformé en base correspondante. On obtient ainsi 3,4 g de produit (rendement 55 %).

Point de fusion F = 154-156 °C

Spectre IR (NUJOL) $\nu$ = 3 000 à 3 400, 1 615, 1 590, 1 550, 1 510, 1 355, 1 170, 1 040, 820, 715, 710 $cm^{-1}$.

Les expérimentations réalisées sur les dérivés du type pyridyl-3 alcoxy (ou phénoxy- ou aralkyloxy)-5 pyrazole conformes à l'invention ont fait apparaître d'intéressantes propriétés pharmacologiques, en particulier une activité inotrope, permettant d'envisager leur application en thérapeutique humaine et vétérinaire comme médicaments cardiotoniques.

La toxité cardiaque des dérivés de formule générale (I) est faible, et on constate par exemple que dans le test sur l'oreillette isolée du cobaye, pour une dose de produit de $3 \times 10^{-4}$ g/ml, aucun trouble de la contraction cardiaque n'est observé.

L'activité inotrope a été mise en évidence sur l'oreillette isolée de cobaye dans les conditions expérimentales suivantes : le liquide de survie est constitué par une solution de Tyrode carbogénée (95 % $O_2$ + 5 % $CO_2$) thermostaté à 30 °C, la tension initiale imposée aux oreillettes est de 0,5 g, le volume de substance à tester additionnée au bain est de 0,5 ml, et le volume du bain à organe de 40 ml ; le temps de stabilisation de l'organe est de 1 heure. La force et la fréquence de contraction sont enregistrées en continu.

A titre d'exemple le (pyridyl-4)-3 méthyl-4 méthoxy-5 pyrazole décrit dans l'exemple 2 procure une augmentation de 120 % de la force de contraction maximale dans les conditions exposées ci-dessus. Ce maximum est atteint en 3 minutes et une augmentation de 25 % de la force de contraction subsiste encore après 60 minutes.

Ces résultats montrent que les dérivés suivant l'invention, de formule générale (I) peuvent être utilisés en thérapeutique, plus particulièrement pour le traitement de l'insuffisance cardiaque.

Les dérivés de formule générale (I) et leurs sels pharmaceutiquement acceptables peuvent être administrés sous les formes usuelles, le principe actif étant dilué dans un support pharmaceutiquement acceptable convenablement choisi, par exemple sous forme de comprimé, gélule, dragée, suppositoire, soluté injectable ou sirop.

A titre d'exemple, les comprimés peuvent être préparés en mélangeant le dérivé de formule générale (I) ou un de ses sels, avec un ou plusieurs diluants solides tels que le lactose, le mannitol, l'amidon, la polyvinylpyrrolidone, le stéarate de magnésium, le talc, etc. Le cas échéant, les comprimés peuvent comporter plusieurs couches superposées autour d'un noyau, suivant les techniques usuelles, pour assurer une libération progressive ou un effet retardé du principe actif. L'enrobage peut par exemple être constitué d'une ou plusieurs couches d'acétate de polyvinyle, de carboxyméthylcellulose ou d'acétophtalate de cellulose.

On peut également administrer le dérivé suivant l'invention sous forme d'un sirop ou d'un soluté buvable obtenu en dissolvant le dérivé de formule (I) ou un de ses sels pharmaceutiquement acceptables, dans de l'eau ou du glycérol, par exemple, et en ajoutant le cas échéant un additif usuel tel qu'un édulcorant et un antioxydant.

Des solutions injectables peuvent être préparées suivant les techniques bien connues et sont constituées par exemple par un soluté contenant un dérivé de formule (I) ou un de ses sels pharmaceutiquement acceptables, dissous dans de l'eau bidistillée, une solution hydroalcoolique, du propylèneglycol, etc., ou un mélange de ces solvants. Le cas échéant, un additif approprié tel qu'un conservateur peut être ajouté.

Les doses administrées sont déterminées par le médecin en fonction du mode d'administration choisi, le niveau de l'affection traitée, la durée du traitement, etc.

## Revendications

1. Dérivés du pyridyl-3 alcoxy (ou phénoxy- ou aralkyloxy)-5 pyrazole représentés par la formule générale (I) :

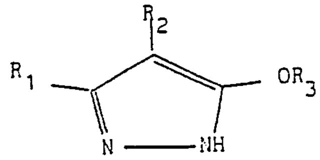

(I)

dans laquelle $R_1$ représente un groupe pyridyl-4, pyridyl-3 ou pyridyl-2 non substitué, ou substitué par un ou deux groupes alkyles inférieurs de 1 à 4 atomes de carbone ; $R_2$ représente un atome d'hydrogène, un

groupe alkyle inférieur de 1 à 4 atomes de carbone, et $R_3$ représente un groupe alkyle inférieur de 1 à 4 atomes de carbone, un groupe phényle ou un groupe aralkyle, ainsi que leurs sels d'addition d'acides.

2. Dérivés selon la revendication 1, caractérisés en ce que $R_1$ est un groupe pyridyl-4, pyridyl-3 ou pyridyl-2.

3. Dérivés selon la revendication 1, caractérisés en ce que $R_2$ est un atome d'hydrogène ou un groupe méthyle.

4. Dérivés selon la revendication 1, caractérisés en ce que $R_3$ est un groupe méthyle, un groupe éthyle, un groupe isopropyle, un groupe phényle ou un groupe benzyle.

5. Dérivé selon la revendication 1, caractérisé en ce qu'il est constitué par le (pyridyl-4)-3 méthyl-4 méthoxy-5 pyrazole ou un de ses sels d'acides.

6. Le (pyridyl-4)-3 benzyl-4 méthoxy-5 pyrazole.

7. Procédé de préparation de dérivés selon la revendication 1, caractérisé en ce qu'on fait réagir une pyridyl-3 pyrazolone-5 de formule (II) :

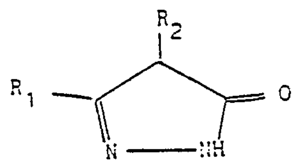

(II)

avec un agent d'alkylation dans un solvant approprié.

8. Procédé selon la revendication 7, caractérisé en ce que l'agent d'alkylation est le diazométhane.

9. Procédé selon la revendication 7, caractérisé en ce que l'alkylation s'effectue par un alcool activé au moyen de triphénylphosphine et d'azodicarboxylate d'éthyle en milieu neutre.

10. Composition pharmaceutique, caractérisée en ce qu'elle comprend un dérivé selon l'une quelconque des revendications 1 à 6, ou un de ses sels pharmaceutiquement acceptables, comme principe actif, associé le cas échéant à un ou plusieurs excipients.

## Claims

1. 3-pyridyl-5-alcoxy or 5-phenoxy or 5-aralkyloxy-pyrazole derivatives represented by the following formula (I) :

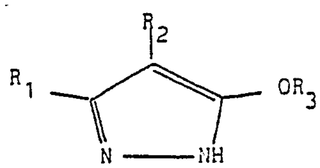

(I)

wherein $R_1$ represents a 4-pyridyl, 3-pyridyl or 2-pyridyl group unsubstituted or substituted with one or two lower alkyl groups with 1 to 4 carbon atoms ; $R_2$ represents a hydrogen atom or a lower alkyl group with 1 to 4 carbon atoms ; and $R_3$ represents a lower alkyl group with 1 to 4 carbon atoms, a phenyl group or an aralkyl group, and the acid addition salts thereof.

2. The derivatives of claim 1, characterized in that $R_1$ is a 4-pyridyl, 3-pyridyl or 2-pyridyl group.

3. The derivatives of claim 1, characterized in that $R_2$ is a hydrogen atom or a methyl group.

4. The derivatives of claim 1, characterized in that $R_3$ is a methyl group, an ethyl group, an isopropyl group, a phenyl group or a benzyl group.

5. A derivative according to claim 1, characterized in that said derivative is 3-(4-pyridyl)-4-methyl-5-methoxy-pyrazole or the acid salts thereof.

6. 3-(3-pyridyl)-4-benzyl-5-methoxy-pyrazole.

7. A process for the preparation of the derivatives of claim 1, characterized in that a 3-pyridyl-5-pyrazolone of formula (II)

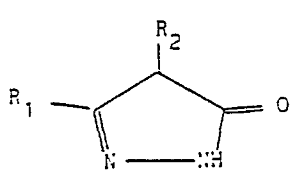

(II)

is reacted with an alkylation agent in an appropriate solvent.

8. The process of claim 7, characterized in that the alkylation agent is diazomethane.

9. The process of claim 7, characterized in that the alkylation is carried out by an alcohol activated by means of triphenylphosphine and ethyl azodicarboxylate in a neutral medium.

10. A pharmaceutical composition characterized in that it comprises a derivative according to any one of claims 1 to 6, or a pharmaceutical acceptable salt thereof, as an active principle, possibly combined with one or several excipients.

**Patentansprüche**

1. 3-Pyridyl-5-alkoxy (oder -phenoxy- oder -aralkoxy) pyrazol-Derivate, dargestellt durch die allgemeine Formel (I)

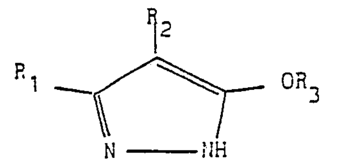

(I)

worin $R_1$ eine 4-Pyridyl-, 3-Pyridyl- oder 2-Pyridyl-Gruppe, die unsubstituiert ist oder substituiert ist durch eine oder zwei niedere Alkylgruppen mit 1 bis 4 Kohlenstoffatomen ; $R_2$ ein Wasserstoffatom, eine niedere Alkylgruppe mit 1 bis 4 Kohlenstoffatomen und $R_3$ eine niedere Alkylgruppe mit 1 bis 4 Kohlenstoffatomen, eine Phenylgruppe oder eine Aralkylgruppe bedeuten, sowie ihre Säureadditionssalze.

2. Derivate nach Anspruch 1, dadurch gekennzeichnet, daß $R_1$ eine 4-Pyridyl-, 3-Pyridyl oder 2-Pyridyl-Gruppe bedeutet.

3. Derivate nach Anspruch 1, dadurch gekennzeichnet, daß $R_2$ ein Wasserstoffatom oder eine Methylgruppe bedeutet.

4. Derivate nach Anspruch 1, dadurch gekennzeichnet, daß $R_3$ eine Methylgruppe, eine Ethylgruppe, eine Isopropylgruppe, eine Phenylgruppe oder eine Benzylgruppe darstellt.

5. Derivat nach Anspruch 1, dadurch gekennzeichnet, daß es besteht aus 3-(4-Pyridyl)-4-methyl-5-methoxy-pyrazol oder einem seiner Säuresalze.

6. 3-(4-Pyridyl)-4-benzyl-4-methoxy-5-pyrazol

7. Verfahren zur Herstellung der Derivate nach Anspruch 1, dadurch gekennzeichnet, daß man ein 3-Pyridyl-5-pyrazolon der Formel (II)

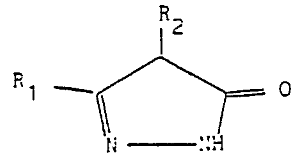

(II)

in einem geeigneten Lösungsmittel mit einem Alkylierungsmittel reagieren läßt.

8. Verfahren nach Anspruch 7, dadurch gekennzeichnet, daß es sich bei dem Alkylierungsmittel um Diazomethan handelt.

9. Verfahren nach Anspruch 7, dadurch gekennzeichnet, daß die Alkylierung in einem neutralen Milieu mit einem Alkohol durchgeführt wird, der mittels Triphenylphosphin und Ethylazodicarboxylat aktiviert worden ist.

10. Pharmazeutische Zusammensetzung, dadurch gekennzeichnet, daß sie ein Derivat nach einem der Ansprüche 1 bis 6 oder eines seiner pharmazeutisch akzeptablen Salze als Wirkstof, ggf. assoziiert mit einem oder mehreren Hilfsstoffen, enthält.